(19) Europäisches Patentamt
European Patent Office
Office européen des brevets

(11) **EP 4 072 717 B1**

(12) **EUROPEAN PATENT SPECIFICATION**

(45) Date of publication and mention
of the grant of the patent:
**22.04.2026 Bulletin 2026/17**

(21) Application number: **20829552.7**

(22) Date of filing: **11.12.2020**

(51) International Patent Classification (IPC):
*B01F 23/232* (2022.01)          *B01F 25/431* (2022.01)
*B01F 25/452* (2022.01)          *B01F 35/00* (2022.01)
*B01J 4/00* (2006.01)          *B01J 10/00* (2006.01)
*B01J 19/18* (2006.01)

(52) Cooperative Patent Classification (CPC):
**B01J 19/1837; B01F 23/2322; B01F 25/4312;
B01F 25/4523; B01F 35/561; B01J 4/002;
B01J 10/002;** B01J 2208/00849

(86) International application number:
**PCT/EP2020/085681**

(87) International publication number:
**WO 2021/116366 (17.06.2021 Gazette 2021/24)**

(54) **STATIC MIXER ELEMENT AND REACTOR COMPRISING A STATIC MIXER ELEMENT**

STATISCHES MISCHELEMENT UND REAKTOR MIT EINEM STATISCHEN MISCHELEMENT

ÉLÉMENT MÉLANGEUR STATIQUE ET RÉACTEUR COMPRENANT UN ÉLÉMENT MÉLANGEUR STATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.12.2019 DK PA201901457**

(43) Date of publication of application:
**19.10.2022 Bulletin 2022/42**

(73) Proprietor: **Unibio A/S
4000 Roskilde (DK)**

(72) Inventors:
• **CHRISTENSEN, Ib**
**3450 Allerød (DK)**
• **ELLESKOV, Michael**
**4490 Jerslev (DK)**

(74) Representative: **Inspicos P/S
Strandvejen 60
2900 Hellerup (DK)**

(56) References cited:
**EP-A1- 1 813 653          DE-A1- 10 126 267
DE-A1- 4 235 979          US-A- 5 327 941
US-A1- 2003 168 754          US-A1- 2011 244 543
US-A1- 2017 205 070**

## Description

The present invention relates to a static mixer element or a reactor comprising the static mixer element for mixing streams of fluid transported through or circulated in a flow reactor, and in particular a static mixer element according to the invention may improve gas/mass transfer by reducing the size of gas bubbles. A flow reactor according to the invention may have any cross-section, e.g. circular, and mixing units are inserted in the flow reactor.

## Background Art:

Static mixers are generally known, and their main advantage is that they have no moving parts and therefore require no driving means and are low on maintenance. Static mixers are particularly suitable for continuously operating plants, where a static mixer may function as continuous-flow mixers.

US patent no. 4062524 relates to an apparatus for the static mixing of streams of fluids such as gases or liquids or more highly viscous substances which apparatus is short in design and not subject to excessively high pressure-loss and yet ensure homogenization. The apparatus comprises a mixing insert comprising at least two plates provided with webs defining slots therebetween the webs of one plate extend crosswise through the slots of at least one other plate which is set at an angle to the first plate. Because of the oblique positioning of the plates, the oncoming stream of fluid is divided by the webs into partial streams the production of which is staggered in time and place, and the partial streams are again subdivided by the line of intersection of the plates. At the back of the webs there is a flow gradient in the transverse direction which produces exchange of the partial stream. Beyond the line of intersection of the pairs of plates, the partial streams are again subdivided, this time in the reverse sequence of time and space. Due to the transverse mixing process, the flow approximates closely to the profile of a so-called plug flow so that a narrow spectrum of dwelling times can be achieved. The inclination of the plates to the direction of oncoming flow and to the wall of the pipe may be multi-dimensional.

US 2011244543 A1 relates to a fermenter and a method of fermentation comprising a U-shape and/or nozzle U-loop fermenter (100). This U-loop fermenter comprises a U-part having an essentially vertical down-flow part (101), an essentially vertical up-flow part (102) and a substantially horizontal connecting part (103) connecting the lower ends of the down-flow part (101) and the up-flow part (102), a top part

(104) which is provided above the U-part and has a diameter which is substantially larger than the diameter of the U-part, means for creating a continuous flow by liquid circulation in the U-part of the fermenter, and one or more gas injection points (110) for the introduction and dispersion of the gas(ses) into the fermentation liquid. The pressure may be controlled differently in certain zones of the fermenter by pressure controlling devices (105, 106, 108) e.g. by increasing the pressure in certain zones of the fermenter in relation to the pressure in the other zones of the fermenter, or decreasing the pressure in a zone of the fermenter in relation to the pressure in another zone of the fermenter in relation to the pressure in another zone of the fermenter. The fermenter may also comprise a number of static-mechanical mixers (13, 14, 15, 16, 17, 116a-116b), each indicating mixing members for dispersion of the supplied gases into numerous small fine bubbles.

US2017056846 A1 relates to a static mixer for mixing two fluids, a first fluid and a second fluid, which two fluids are two miscible components of a single-phase flow. The static mixer including a plurality of plates having orifices formed therethrough. As the first fluid and the second fluid pass through the static mixer, the fluids are combined and mixed.

KR2017009397 relates to an apparatus for generating fine bubbles in liquid. The apparatus comprises an injection unit including a liquid injection pipe connected to an injection opening 110 of a chamber 100. The apparatus may further comprise a gas injection tube 320 for injecting gas into the liquid injection tube 310. The liquid may be pumped from a reservoir by a pump and supplied to an inner space of the chamber 100 through the liquid injection tube 310. The chamber 100 represents an increase in cross-section compared to the liquid injection tube which means that the pressure is decreased and the air/oxygen in the liquid may be released as bubbles. In the case where only liquid is injected microbubbles may be generated by cavitation, and then only the liquid needs to be injected through the injection tube (See paragraph [0026]). I.e. this apparatus is suitable for working at relatively low pressures, e.g. pressures below 1 bar inside the chamber 100.

US 2017/0205070 A1 discloses a static mixer element according to the preamble of claim 1.

The apparatus according to the present invention is suitable for working under a relatively high pressure, i.e. a pressure above atmospheric pressure, where the static mixer element makes it possible to mix a gas comprising a component of low solubility at atmospheric pressure into the liquid, e.g. for mixing methane into water.

The object of the present invention is to provide a more compact, more economic and even more efficient static mixer configured to mixing a gas into a liquid by reducing bubble size.

## Summary of invention:

According to a first aspect of the invention, the invention relates to a static mixer element for installation in a flow channel according to claim 1.

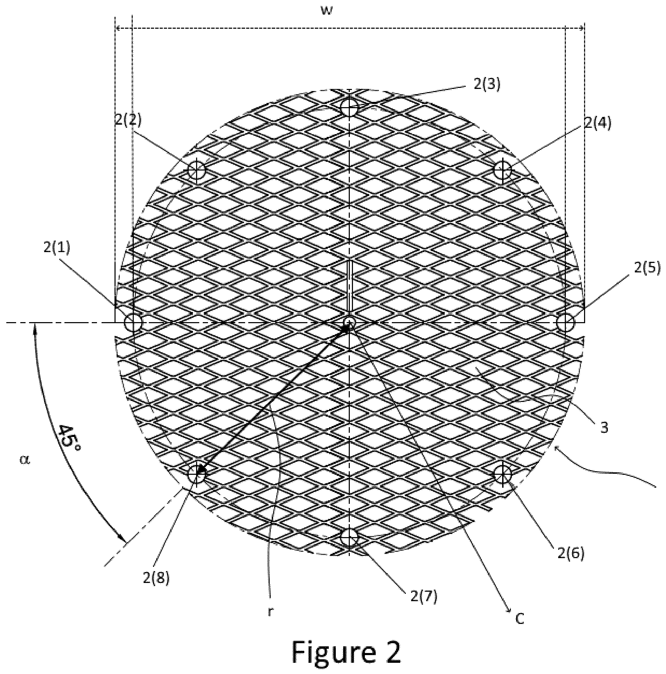

According to any embodiment of the first aspect, the plurality of through openings (3) may be distributed evenly across the surface of the mixer element facing the flow direction. That the openings are "distributed evenly" means that each opening is positioned with same or approximately same distance to its neighboring openings, or that the openings are positioned in a uniform pattern covering the surface or at least half or two-thirds of the surface. Figure 2 shows a uniform pattern of rhombuses distributed over the complete surface of a mixer unit; however, the openings may also have a round or rectangular cross-section or the openings may be distributed only over a part of the mixer unit surface, e.g. over half the surface or over 2/3 of the surface.

According to any embodiment of the first aspect of the invention, the static mixer may comprise at least two mixer units, e.g. exactly or at least 3, 4, 5, 6, 7, 8, 9, 10 or more mixer units, or between 2 and 100 mixer units, or between 2 and 50 mixer units or between 4 and 40 mixer units or between 4 and 35 mixer units, where each through opening in each previous mixer unit at least partly faces a surface of the following mixer unit thereby forcing the fluid flow to change direction.

According to any embodiment of the first aspect of the invention, each mixer unit may form a fluid-tight connection to or lie against an inner surface of the flow channel.

According to any embodiment of the first aspect of the invention, the thickness or height h of a mixer unit may be significantly smaller than the cross-section or width w of the mixer unit (1), i.e. $f_{min} \leq \frac{w}{h} \leq f_{max}$, where $f_{min} \leq 10$ or 20 or 40, and $f_{max} \geq 100$ or 150 or 200.

According to any embodiment of the first aspect of the invention, the thickness of a mixer unit in areas comprising through openings may be between 2 - 60 mm, or 2 - 40 mm, or 2 - 30 mm or 2 - 15 mm.

According to any embodiment of the first aspect of the invention, a previous and a following mixer unit are substantially parallel and may normally comprise a surface facing perpendicular to the direction of flow, i.e. a surface of a first mixer unit facing a surface of a following mixer units may deviate less than 10°, normally less than 5° or less than 2° from the following mixer unit (1).

According to any embodiment of the first aspect of the invention, there may be a distance d between a surface of a previous mixer unit facing a surface of a following mixer unit, the distance d may be less than 10 times or less than 5 times the thickness or height h of the previous mixer unit, or e.g. the distance d may be less than 2 times the thickness or height h of the previous mixer unit.

According to the invention, the mixer units are positioned in and attached to a frame, which frame may define a position for each mixer unit relative to other mixer units in a static mixer element.

According to the invention, each mixer unit comprises position lockers, the position lockers are distributed equally, i.e. with same radial distance between adjacent position lockers measured in degrees (°), along a circle with radius r and center C.

According to any embodiment of the first aspect of the invention, the through going openings in each mixer unit may be of same shape and size and the shape of the through going openings may be e.g. rhombic or circular or rectangular.

According to a second aspect of the invention, the invention relates to a static mixer section comprising one or more blocks of static mixers where at least one block of static mixers has the function of distributing gas bubbles over the flow cross-section and at least one block constituted of two or more static mixer elements according to the first aspect of the invention.

According to any embodiment of the second aspect of the invention, the at least one block of static mixers having the function of distributing gas bubbles over the flow cross-section may be positioned upstream relative to a static mixer element according to the first aspect.

According to a third aspect of the invention, the invention relates to a reactor according to claim 8 and one or each static mixer element of the reactor may be positioned in a flow channel or in a tubular part of the reactor comprising a cavity which cavity comprises an inlet opening and an outlet opening. The size of the cross-sectional area of the flow channel or tubular part of the reactor may be relatively constant compared to the inlet of the flow channel or tubular reactor, where "relatively constant" means that the size of the cross-sectional area varies less than 10%, e.g. less than 5% from the size of the inlet. As the cross-sectional area is relatively constant before and inside a reactor chamber, the pressure does not decrease suddenly.

Also, the reactor may be configured to be operated at a pressure above 1 bar, e.g. above 2 bar, or above 3 bar.

According to any embodiment of the third aspect of the invention, the reactor may be a loop reactor.

According to any embodiment of the third aspect of the invention, the reactor may be a fermentation reactor comprising a loop-part and a top tank, said loop-part comprising a downflow part, connected to an up-flow part via a U-part, wherein the top tank comprises:

(i) a first outlet connecting the top tank to the downflow part of the loop-part and allowing a fermentation liquid present in the top tank to flow from the top tank into the loop-part;
(ii) a first inlet connecting the top tank to the up-flow part of the loop-part, allowing fermentation liquid present in the loop-part to flow from the loop part into the top tank; and

(iii) a vent tube for discharging effluent gasses from the top tank;

where at least one static mixer element is positioned in the loop part.

According to any embodiment of the third aspect of the invention, more than one static mixer element i.e. at least two static mixer elements may be positioned in the loop part, e.g. exactly or more than 2, 3 or 4 static mixers are positioned in a loop part of the reactor.

According to a fourth aspect of the invention, the invention relates to use of a static mixer element according to the first aspect in a reactor for mixing a two-phased flow comprising a liquid and a gas.

According to any embodiment of the fourth aspect of the invention, the gas is $O_2$ and / or the liquid is a fermentation liquid.

**List of figures:**

Figure 1 discloses an embodiment of a U-shaped reactor in which a static mixer element according to the invention may be employed.

Figures 2 discloses an embodiment of a mixing unit of a static mixer element according to the invention.

Figures 3A and 3B disclose a frame for positioning between mixing units.

Figure 4 discloses a first embodiment of a static mixer element according to the invention.

Figure 5 discloses a second embodiment of a static mixer element according to the invention.

Throughout the application identical or similar elements of different embodiments are given the same reference numbers.

**Definitions of words:**

*In general-* when this expression is used in respect of a feature, the feature may be employed with any embodiment of the invention, even though the specific mentioning appears in the detailed part of the description.

*Static mixer* - a mixer without moving parts.

*Flow reactor* - a reactor where a continuous flow is created at least in a part of the reactor during operation.

**Detailed description of invention:**

The present invention relates to a static mixer element comprising two or more mixer units 1. A static mixer element according to the invention provides mixing of liquids and homogenization of liquid/gas mixtures by minimizing or reducing the size of bubbles in the liquid phase.

Fig. 2 discloses a view in the flow direction of an embodiment of a mixer unit 1 which may be applied in a static mixer element according to the invention.

In general, one mixer unit 1 comprises a plurality of through openings 3 and one or more position lockers 2, normally, the through openings 3 are identical i.e. all through openings 3 of one mixer unit 1 have same shape and size. Further, the through openings 3 may be distributed evenly across the complete flow area of the mixer unit 1 where the flow area is the cross-sectional area where fluids may flow during operation. The through openings 3 allows liquid to pass through the mixer unit 1 and the position lockers 2 are openings or protrusions or similar which lock or fix the radial movement of a first mixer unit 1 relative to a second and adjacent mixer unit 1.

The mixer unit 1 has the overall shape a plate or a sheet i.e. the thickness or height h of the mixer unit 1 is significantly smaller than the cross-section or width w of the mixer unit 1, i.e. normally $f_{min} \leq \frac{w}{h} \leq f_{max}$. The height h of a mixer unit may be between 0.002 and 0.10 m, normally between 0.002 and 0.05 m, e.g. between 0.002 and 0.015 m. The width of a mixer unit w i.e. the maximum cross-sectional dimension will be adapted to the line of the flow reactor in which the mixer unit 1 is placed, and may be between 0.20 and 2.5 m, normally between 0.30 and 2.0 m, e.g. between 0.3 and 1.0 m. E.g. $f_{min} \leq 10$ or 20 or 40, and $f_{max} \geq 100$ or 150 or 200 (Example: $\frac{w}{h} = \frac{392}{8} = 49$, or $\frac{w}{h} = \frac{1000}{3,5} = 286$ ).

Also, the thickness/height h of the mixer unit 1 and the surfaces in the cross-sectional plane are relatively flat in areas comprising through openings 3, i.e. normally the thickness or height h of a mixer unit 1 deviates less than 10%, e.g. less than 5%, e.g. less than 1% throughout the cross-sectional plane comprising through openings 3, i.e.

$$\frac{h_{max} - \overline{h_{min}}}{h_{max}} \leq 0,10$$

or 0,05 *or* 0,01. The height h may vary more in areas comprising position lockers 2 i.e. position lockers 2 may extend from the surface of the mixer unit 1. Normally, a previous and a following mixer unit 1 are substantially parallel, i.e. the angle between a surface of a first mixer unit 1 and a surface of a second or adjacent mixer unit facing each other deviates less than 10°, normally less than 5° or less than 2° from the following mixer unit 1.

As adjacent mixer units 1 are substantially parallel the distance d between two adjacent mixer units 1 is normally substantially constant. E.g., the distance between adjacent mixer units 1 in a static mixer element is between 0.5 the thickness of a mixer unit 1, i.e. 0.5xh, and 3 times the thickness of a mixer unit 1, i.e. 2xh. E.g. the distance between adjacent mixer units may be at least 0.001 m, or at least 0.003 mm, or at least 0.006 m, and/or at maximum 0.030 m, or at maximum 0.020 m or at maximum 0.010 m.

The outer periphery of a cross-sectional plane of a mixer unit 1 corresponds to inner surfaces of a flow channel in a part of a reactor where a static mixer element is to be positioned. I.e. either each mixer unit 1 or the static mixer element as such touches the inner surfaces of the reactor, or each mixer unit 1 or the static mixer element as such may be provided with fitting parts or an adapter or packings extending between the inner surfaces of the reactor and the outer perimeter of each mixer unit 1 or the outer perimeter of the static mixer element as such thereby preventing fluid from bypassing any of the mixer units 1 or the static mixer element as such.

According to the embodiment shown in fig. 2, the perimeter of the shown mixer unit 1 is circular. In general, the perimeter of each mixer unit 1 may be circular or oval, however, the perimeter of a mixer unit 1 may have any shape e.g. and the perimeter of a mixer unit 1 may be adapted to another inner surface profile by a packing or similar.

The relatively small dimension of each mixer unit 1 in the overall flow direction i.e. h, makes it suitable to combine a number of mixer units 1 into a static mixer element having a customized pressure loss and mixing ability. To reduce costs, it is advantageous that the mixer units 1 constituting the mixer units of a static mixer element are either identical or substantially identical.

According to the invention, a mixer unit 1 comprises a plurality of position lockers 2, the plurality of position lockers is constituted of through going openings, e.g. identical through going openings 2, allowing insertion of a rod in the through going openings 2 thereby preventing relative radial movement of the mixer units 1, where a radial movement is motion at a right angle to an axis of rotation. Essentially, a radial movement is movement around a shaft rather than along the length of the shaft.

It is possible to control the mixing ability of a static mixer element constructed by a plurality of identical mixer units 1 by providing a first or previous mixer unit 1 and a second or following mixer unit 1, which second or following mixer unit 1 is displaced radially relative to the first or previous mixer unit 1 in a direction of flow, in such a way that an opening in the first or previous mixer unit 1 at least partly faces a surface of the second or following mixer unit 1, thereby forcing the fluid flow to change direction.

A static mixer element may be constructed by positioning a number of mixer units in a series of e.g. between 4 and 50 adjacent mixer units 1 in the direction of flow so that fluid to be mixed flows through all mixer units and displacing the mixer units 1 radially relative to each other. By constructing such a static mixer element, it is possible in a simple and cost-effective way to control degree of homogenization relative to pressure loss in a flow reactor, and it is also possible to improve the gas/mass transfer i.e. transfer of components from gas to liquid by reducing the size of gas bubbles.

According to one embodiment, each mixer unit 1 in a static mixer element comprises at least four position lockers 2, or e.g. at least eight position lockers 2, and normally all mixer units 1 in one static mixer element 2 is provided with the same number of position lockers 2.

The position lockers 2 of a mixer unit 1 are distributed equally, i.e. with same radial distance between adjacent position lockers 2 measured in degrees (°), along a circle with radius r and center C, such that it is possible to position each mixer unit 1 with a radial displacement relative to a formerly placed mixer unit 1 simply by turning the following mixer unit 1 relative to the previous mixer unit 1.

The embodiment shown in fig. 2 comprises eight position lockers 2: 2(1), 2(2), ..., 2(8) distributed equally along a circle with radius r and center C, the radial distance between adjacent position lockers is 45°. A first mixer unit 1 in a static mixer element comprising a series of this type of mixer units may be positioned as shown in fig. 2, a second and adjacent mixer unit 1 may be turned relative to the first mixer unit 1, in such a way that the position locker of the second mixer unit 1 corresponding to 2(1) of the first mixer unit 1 is placed at the position 2(2) of the first mixer unit 1, and the second mixer unit is then radially displaced 45° relative to the first mixer unit 1. A third mixer unit 1 may then again be radially displaced 45° relative to the second mixer unit 1 and 90° relative to the first mixer unit 1.

If an embodiment comprises three position lockers distributed equally along a circle with radius r and center C, the radial distance between adjacent position lockers is 120°.

The through openings 3 of a mixer unit 1 according to the invention may have any shape or form, preferably the through openings 3 are shaped or formed as parallelograms e.g. squares, rectangles or rhombuses.

The mixer units 1 may be prepared from metal such as stainless steel or similar. Alternatively, the mixer units 1 may be prepared from a polymer or the mixer units 1 may be prepared from a ceramic material. The mixer units 1 may be prepared from an expanded metal, where a metal sheet is first provided with parallel cuts and then extended in a direction perpendicular to the cuts. Expanded metal will provide parallelogram shaped through openings 3. Alternatively, it will be possible to cut or stamp through openings 3 directly into a metal sheet.

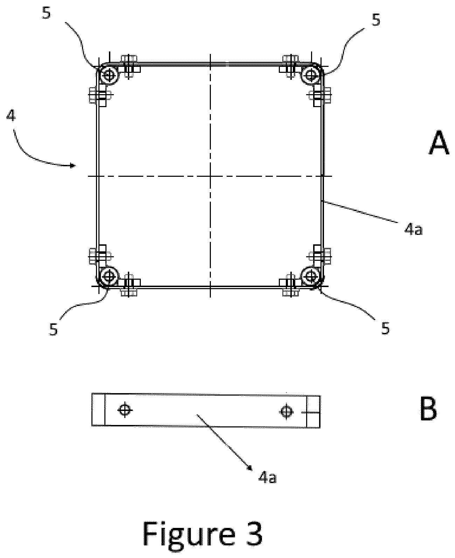

Fig. 3 discloses an embodiment of a frame 4 which is used to fix the position of a plurality of mixer units 1, e.g. a plurality of mixer units 1 of an embodiment as shown in fig. 2. This type of frame 4 may be positioned at one or at both ends of the static mixer element.

The frame 4 comprises a part 4a extending in the overall flow direction which part 4a is long or high enough to provide a stable support for a static mixer element. Further, the part 4a may constitute a closed perimeter, e.g. a square as shown in the embodiment of fig. 3, however, the perimeter of the part 4a may alternatively be circular or oval and the outer perimeter of the frame 4 is smaller than the perimeter of the mixer units 1.

The frame 4 is combined with rods 5 extending in the full length of a static mixer element. The rod is fastened to the frame by fastening means 8. The length of the rods 5 is adapted to the numbers of mixer units 1 which defines the length of a static mixer element. The rods 5 may be fixed to a frame 4 or they may be replaceable. The rods may be made of metal or polymer or ceramic material. The rods 5 fit into openings constituting position lockers 2 in the mixer units 1 of a static mixer element.

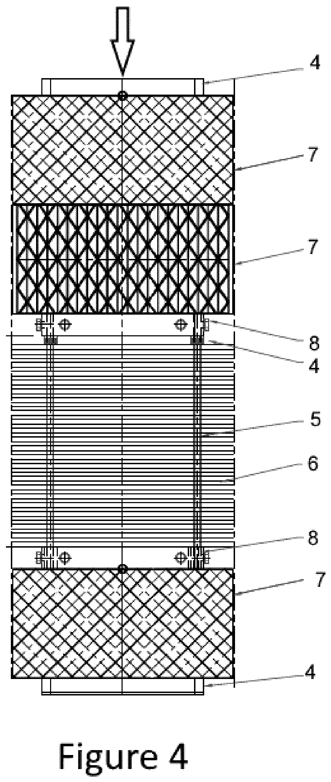

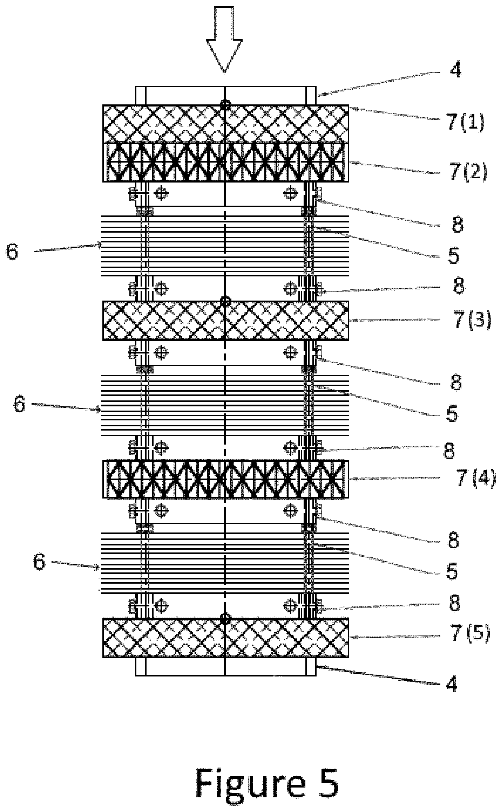

A static mixer element according to the invention may be combined with other static mixer elements according to the invention and/or be combined with known static mixer blocks. Examples of such combinations are shown in fig. 4 and 5.

Fig. 4 shows an embodiment comprising 1 static mixer element comprising 45 mixer units 1 according to the invention in combination with 3 other mixer blocks.

This embodiment is also an example of a static mixer section according to the invention where one or more block comprising a number of static mixer elements according to the present invention, e.g. between 2 and 100 static mixer elements, is combined with one or more blocks of another type of static mixing units e.g. static mixing units known according to prior art. The other types of mixing unit are primarily used for distributing gas bubbles evenly over the complete cross-section of the pipe in which the static mixer block is positioned and may therefore be positioned upstream of the block of static mixer element according to the invention. The block of static mixer element according to the invention contributes to distribution of gas bubbles but will also cause a reduction in the size of the gas bubbles resulting in improved gas/mass transfer.

The embodiment disclosed in fig. 4 comprises in the direction of flow indicated with an arrow at top of the section, a first block 7(1) of a first type of static mixer which contribute to distributing gas bubbles evenly across the flow of gas/liquid mixture, a second block 7(2) of a second type of static mixer which also contribute to distributing gas bubbles evenly across the flow of gas/liquid mixture, a third block constituted of static mixers elements 6 according to the invention which primary contribution is breaking down gas bubbles, and a fourth block 7(3) of the first type of static mixer which contributes to distribute the smaller gas bubbles evenly over the cross-section of the pipe in which the static mixer section is positioned.

Fig. 5 shows an embodiment comprising 3 static mixer elements each comprising 13 mixer units 1 according to the invention in combination with 5 different mixer blocks.

The embodiment disclosed in fig. 5 comprises in the direction of flow indicated with an arrow at top of the section, a first block 7(1) of a first type of static mixer contributing to distributing gas bubbles evenly across the flow of gas/liquid mixture, a second block 7(2) of a second type of static mixer also contributing to distributing gas bubbles evenly across the flow of gas/liquid mixture, a third block constituted of static mixers elements 6 according to the invention primary contributing to breaking down gas bubbles. Further, the embodiment comprises a fourth block 7(3) of the first type of static mixer, then a fifth block static mixers elements 6 according to the invention, a sixth block 7(4) of the second type of static mixer, a seventh block of static mixers elements 6 according to the invention and at last an eight block of static mixers of the first type.

When constructing a static mixer section as exemplified in figs. 4 and 5 the types of static mixers and the number of static mixer blocks may be adapted to the specific mixing process and mixing apparatus, i.e. according to any embodiments of a static mixer section the number of static mixer blocks may be varied and the type of static mixer blocks may be varied.

**Example of reactor having an internal flow:**

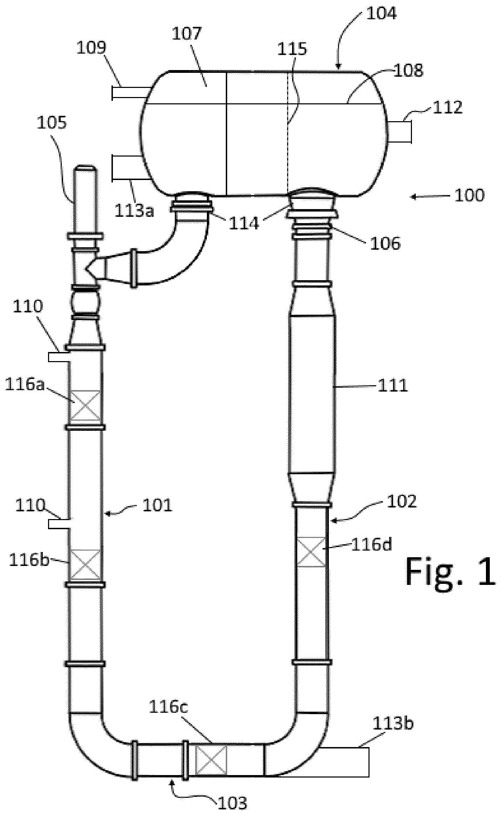

Fig. 1 shows a reactor having continuous flow, this type of reactor may be referred to as a fermenter. The fermenter is a U-shaped fermenter 100 comprising a top part 104 and a U-part, which U-part comprises two legs, a down-flow leg 101 and an up-flow leg 102 and a horizontal connecting part 103 connecting the two legs.

The cross-section of the legs 101, 102 and the horizontal connecting part 103 is normally substantially circular or oval, but the cross-section may be polygonal, e.g. square, rectangular or hexagonal. The height of the legs in relation to the length of horizontal connecting part 103 (height legs : length lower connecting part) may be in the range between 100:1 to 1 :100, preferably between 50:1 to 1 :50 or 25:1 to 1 :25, the most preferred ratio being 10:1. The overall volume of the fermenter may be in the range of 0,001 m$^3$ to 1000 m$^3$, e.g. in the range of 0.2-500 m$^3$, or e.g. in the range of 50-250 m$^3$. The diameter of each legs and the lower horizontal connecting part in relation to the height of the legs will depend on the overall volume of the fermenter needed, and may be calculated by any person skilled in the art.

A fermenter may comprise one or more static mixers and optionally a dynamic mixer, the embodiment shown in fig. 1 comprises four static mixers 116a-116d. The number of mixers and the position of the mixers are determined by the need to modify mixing or to modify the size of the gas bubbles in the liquid flowing through the fermenter. Mixers 116a- 116d may be placed in one or in both legs 101, 102 of the fermenter, e.g. a mixer may be positioned in the down-flow leg 101 down-stream of where gas is added as gas-addition will increase the need for mixing, or a mixer may be placed at any position in the fermenter legs where gas redistribution is needed, e.g. due to increased coalescence of the gas bubbles or decreased hydrostatic pressure in the liquid.

The number of mixers may be varied according to the need for gas dispersion in the fermenter and is not limited to the number or places of the mixers 116a-116d shown in the fig. 1. The need for gas dispersion in a fermenter e.g. depends on the microorganisms used and the concentration of microorganisms as well as the composition and/or physical properties (e.g. temperature, viscosity, etc.) of the broth.

The upper parts of the two legs, i.e. a down-flow leg 101 and an up-flow leg 102, are connected by a top part 104 which top part 104 connects the flow between the down-flow and the up-flow legs. The fermenter may be completely closed from the surroundings except for pipes and fittings (not shown) necessary to insert sensors or to add or remove substances to the fermenter. The top part 104 is normally a container having a cylindrical or oval cross-section, and normally the top part 104 has a larger diameter than the legs of the fermenter. The cross-section of the top part 104 does not need to be cylindrical or oval, but may also be of polygonal shape, e.g. rectangular, square or hexagonal. The top part 104 is connected with straight or conical connection parts 114 for connecting the top part 104 to the legs 101, 102. The top part 104 of the fermenter is normally designed to contain a substantial part of the fermentation liquid. The top part 104 may for example be designed to contain between 1 % and 99 % of the overall volume of the fermenter, but normally between 10 % and 60 % of the overall fermenter volume. It may be preferred that the volume of the top part is less than the volume of the U-part. When the fermenter is in operation, the top part 104 may be filled completely or partly with liquid. E.g., the fermenter legs 101 and 102 and the connecting part are flooded completely and the top part 104 is flooded partially with fermentation liquid, leaving a part of the volume, a headspace 107, in the top part 104 above a liquid surface 108 available for collection of gases. The size, i.e. both the diameter and the height of the fermenter may vary according to the needs of total fermenter volume.

The top part 104 may comprise liquid or gas flow modifying means 115 to assist mixing in the top part 104 or to assist gas bubble release from the liquid. The gas or liquid flow modifying means 115 may e.g. be baffles or static mixers.

One or more pressure controlling devices may be placed in the fermenter to permit the pressure in the fermenter to be controlled, such that different zones of the fermenter experience a higher or lower pressure than other zones. One or more of the devices used to control the pressure may also be used to circulate the liquid and/or the gas-liquid mixture inside the fermenter.

A first pressure controlling device 105 may be placed near the top of the down-flow leg 101. The first pressure controlling device 105 may circulate the liquid in the fermenter, and at the same time, cause an increase in pressure when the liquid or gas-liquid mixture passes through the first pressure controlling device. The first pressure controlling device 105 may be a pump, e.g. a propeller pump, a lobe pump or a turbine pump especially designed for circulating a gas- liquid mixture. Other suitable means for increasing the pressure and creating liquid circulation in the fermenter are e.g. addition of a pressurized gas, e.g. air or an inert gas in combination with a liquid circulating device, which may be a pump.

A second pressure controlling device 106 may e.g. be placed in one of the legs 101,102 or in the horizontal part of the U-bend 103, such that the pressure may be increased between the pressure controlling devices 105 and 106 when seen in the flow direction. When the pressure is increased in a zone in the fermenter, the solubility of the injected gases in the liquid phase is also increased. E.g., the second pressure controlling device 106 may be placed in the middle to the top of the up-flow leg 102. The pressure controlling devices 105,106 may be operated such that the pressure in the fermenter in a first zone down-stream of the first pressure controlling device (105-103-106) is increased to a pressure above the pressure in a second zone down-stream of the second pressure controlling device (106-104-105) which first zone may include the horizontal connecting part 103 and which second zone may include the top part 104.

If the entire fermenter is operated at a pressure above atmospheric pressure, the pressure controlling devices 105,106 may also be operated such that the pressure in the fermenter in the second zone (106-104-105) is decreased to a pressure below the pressure in the first zone (105-103-106) e.g. by connecting a vacuum suction line (not shown) connected to the headspace 107 above the liquid surface 108 of the fermenter.

The pressure controlling device 106 may be chosen among a number of pressure controlling devices such as: a narrowing of the diameter/cross section of the leg 102 or of the connecting part 103, a plate with holes, jets or nozzles inserted in the up-flow leg 102 or the horizontal connecting part 103, a valve controlled by the pressure at one or more locations in the fermenter, a static mixer, a hydro cyclone or a pump, such as a propeller pump, a lobe pump or a turbine pump.

Static mixers are normally optimized in respect of mixing, while the pressure drop through the mixers is minimized. If a static mixer is used as a pressure controlling device, the static mixer may be constructed to optimize pressure drop according to a desired pressure drop required in the fermenter. Alternatively, the second pressure controlling device 106 may be a propeller pump. The propeller pump may be driven by a motor if it is to increase the pressure, e.g. due to pressure loss through the static mixers 116.

Alternatively, the propeller pump is connected to a device restricting the rotation and thereby decreasing the pressure. A device which restricts rotation may be a brake, or, alternatively, it may be a generator which is capable of collecting some of the energy used to increase the pressure in the fermenter. The collected energy may be re-used at other locations of the system, thus reducing the overall energy consumption of the system. In another embodiment of the invention, the pressure controlling device 106 is either a narrowing of the diameter/cross-section of the up-flow leg 102 or the horizontal connecting part 103 or a plate with holes, jets or nozzles. If necessary, a third pressure controlling device may be placed elsewhere in the fermenter, e.g. after i.e. down-stream of the second pressure controlling device 106 and up-stream of the first pressure controlling device 105. The pressure may be decreased in a third zone between the second and the third pressure devices in respect to the pressure in the first zone between the first pressure controlling device 105 and 106, i.e. the pressure is reduced in two steps before the broth enters the top part 104 of the fermenter. The reduced pressure in the third zone may aid coalescence of gas bubbles and further assist in release of waste gases, such as $CO_2$, from the fermentation liquid in the top part 104. The third pressure controlling device may be of the same types as described for the second pressure controlling device 106 and is e.g. also a narrowing of the diameter/cross-section of the leg 102 or the connecting part 103 or a plate with holes, jets or nozzles or a static mixer.

The fermenter may comprise one or more injection points 110 for adding gaseous substances directly to the broth in the fermenter, and the fermenter may also have injections points for adding liquid substances. The gaseous substances may be added anywhere along the legs 101,102 or the horizontal connecting part 103 of the fermenter. The added gas may e.g. be pure oxygen, sterilized air or sterilized air enriched with oxygen added through injection points 110 in the top of the down-flow leg 101. The injection points 110 may be placed closely after the pressure controlling device 105 i.e. downstream of the device 105. At this location in the fermenter, the hydrostatic pressure only contributes slightly to the total pressure in the liquid. Therefore, the need for compression of the gas, e.g. air or oxygen, is reduced to a minimum or even eliminated, which reduces the overall energy consumption of the system. Other gases, such as methane or ammonia may be added in the same way, e.g. in mixture with the air/oxygen through injection points 110 or through other nozzles (not shown) placed in the down-flow leg 101, the up-flow leg 102 or horizontal connecting part 103. The injection points 110 for addition of gases may be single-flow nozzles and/or multi-flow nozzles. Single-flow nozzles add the gases directly to the broth, and multi-flow nozzles are e.g. jet nozzles driven by a pump, which recirculates liquid taken from elsewhere in the fermenter, optionally via a holding tank, and mixes liquid with gases before or during injection into the fermenter. Alternatively, the nozzle arrangement may comprise several nozzles of different construction, each being optimized for gas addition in different periods of the fermentation process. The nozzle arrangement for gas addition may e.g. comprise both single-flow and multi-flow nozzles, where the single flow nozzles are primarily used during start and initial fermentation, followed by the use of multi-flow nozzles during steady state fermentation. The nozzles for gas addition may optionally be arranged in a manifold-like construction. Additional gaseous substrate or other gaseous substances may be also be added at one or more locations of legs and horizontal connecting part of the fermenter. If the gases are added at one or more additional injection points, static gas mixers are normally placed in connection with i.e. just downstream of the gas injection points.

Liquid substances, e.g. make-up water, aqueous solutions of substrate components, pH-regulating solutions may be added at one or more locations along the legs and horizontal connecting part of the fermenter, especially if one or more of the substrates acts as a catabolic inhibitor to the microorganisms used. An outlet 113a, 113b for transferring broth to downstream processing equipment may be placed in the top part 104 and/or in the horizontal connecting part 103. A first outlet 113a may preferably be placed in the top part 104 when the fermenter is run in continuous fermentation mode. Alternatively, or in addition to the first outlet 113a, a second outlet 113b may be placed in the horizontal connecting part 103, i.e. the lowermost part of the reactor. The second outlet 113b may be used when the fermenter is emptied entirely, e.g. for inspection or for sterilization after a period of fermentation. The second outlet 113b is the primary outlet if the fermenter is run in batch mode or fed batch mode.

The pressure in the headspace 107 may be controlled by any known procedure, e.g. by releasing gas from headspace 107 through one or more valves (not shown) connected to an outlet.

The headspace 107 may be flushed with a flushing or stripping gas to assist in the removal waste gases such as $CO_2$ or other gases produced during fermentation, or other gases present in the headspace 107. Flushing of the headspace 107 is also relevant if methane is present in critical amounts in order to reduce or eliminate any risk of explosion. The flushing gas may be air, nitrogen, carbon dioxide itself or any combination of known gases suitable as a flushing gas. The flushing gas is added to the headspace through a device i.e. an inlet 109, such as a pipe and/or one or more nozzles in such a way that it flushes the liquid surface 108 and/or the headspace 107 and leaves the top part through an outlet placed above surface 108. The flow of the flushing gas may flush the liquid surface and or headspace co-currently, con-currently or cross-currently in respect to the direction of the liquid flow in the top part. Normally, a counter-current flow of flushing gas is used. The position of the inlet 109 and the outlet for flushing gases is adjusted according to the flow direction of the flushing gases. Alternatively, or in addition to flushing of the headspace, one or more gaseous substances may also be added below the liquid surface in the top part 104 of the fermenter. If the flushing gas, such as air or an inert gas, is added at the bottom of or at the inlet to the top part 104, it may assist in stripping off any rest of solubilized waste gases, especially CU2, from the fermentation liquid and into the headspace 107.

The U-part of the fermenter may be equipped with a temperature regulating device 121 for heating or cooling the circulating fermentation broth. The temperature regulating device 121 is normally a cooling and/or heating jacket mounted at the upper part of the up-flow leg 102 as shown on fig. 1, having fittings for circulating heating or cooling media through the jacket. Alternatively, the heating/cooling jacket may be mounted on the down-flow leg 101. If needed, the fermenter may be equipped with more than one temperature adjusting device or heating/cooling jacket, e.g. both on the down-flow leg 101 and the up-flow leg 102.

One or more pressure sensing devices (not shown) are placed inside the fermenter. Preferably, at least one pressure sensor is placed in each zone of the fermenter where it is desired to maintain a particular pressure. One or more pressure sensing devices may be connected to a process control system, e.g. comprising a computer, which may control the pressure controlling devices 105, 106, 108 in order to maintain an optimal pressure in each zone of the fermenter. One or more sensors (not shown) for determining dissolved oxygen (DO) may also be placed in the fermenter in order to detect if the oxygen level in the fermenter is kept within a predefined range, which depends on the microorganism or microorganisms used in the fermentation.

Additional sensors, e.g. for measuring temperature, pH, conductivity measurements, oxidation reduction potential and different ions present in the broth, e.g. ammonia, nitrite, nitrate, phosphates, etc., may be present in the circulating broth and/or the recirculated supernatant from down-stream processing. The sensors may be commercially available sensors and may include biosensors, electrochemical sensors, e.g. ion sensitive electrodes or sensors based on FIA (flow injection analysis) and optical measurements, e.g. spectrophotometric devices. A Near Infrared (NIR) probe may be used for measuring several different components in the broth or in the cells in the fermenter, e.g. concentration of cells, amino acids, methanol, ethanol and/or different ions. The fermenter may be equipped with a mass spectrometric (MS) sensor or an electronic nose for determining the concentration of gaseous and volatile components (e.g. $CO_2$ and/or $CH_4$) in the headspace 107. The MS sensor or the electronic nose may control the pressure applied in the fermenter and/or the addition of gaseous components, e.g. methane and/or air/oxygen and/or the addition of gaseous ammonia or the ammonia/ammonium in solution. A high-speed camera may be installed in the U-part of the fermenter, e.g. in connection with gas injection, for determining the bubble size of the gases in the broth. The bubble size may be determined by image processing of the data from the high-speed camera.

Recirculated supernatant may be added to the top part of the fermenter via an inlet 112, or it may be added at one or more positions in the U-part of the fermenter. Return of supernatant from downstream processing reduces the overall consumption of substrates, carbon and minerals, thus reducing the costs of the fermentation process.

The connection parts 114 may or may not contain vortex hindering means (not shown), e.g. baffles or the like according to needs.

The fermenter may be run in continuous operation mode, the continuous operation mode is started after a cleaning and sterilization procedure followed by a start-up period in which water, necessary nutrient salts and microorganisms are added to the fermenter, then the fermentation broth is circulated in the fermenter, e.g. by the first pressure controlling device 105. During start-up addition of gaseous substrates is initiated and fermentation starts.

When microorganisms have reached a certain level of concentration, e.g. a concentration of approximately 0.5-10 %, and preferably 1-5 % (by dry weight), the fermenter enters continuous operation mode where fermentation broth is continuously withdrawn from the fermenter for downstream processing. Withdrawing of fermentation liquid through the outlet 113a is normally initiated simultaneously with addition of e.g. make-up water, aqueous substrate and/or recirculation of supernatant e.g. through the inlet 112 at a dilution rate depending on the microorganisms used in the fermentation. If M. Capsulatus is used, the dilution rate is e.g. 0.01 - 1.5 $h^{-1}$ and preferably between 0.2 - 0.8 $h^{-1}$. Addition of substrate components in liquid solution, additional water, recirculation of supernatant as make-up for the withdrawn broth and substrate gases are controlled by a unit such as a computer receiving data from the gas sensors in the headspace, ion sensors or analyzers, thermo sensors, pressure sensors, etc. and calculating the necessary amounts of each component for obtaining optimized growth of the microorganisms. Hydraulic circulation time in the fermenter is normally around 10-15 seconds depending on the size of the fermenter and the effect of the first pressure controlling device.

According to one embodiment of the method, the fermenter may be divided into two zones by at least two pressure controlling devices 105,106 which two zones are maintained at different pressures. The first fermenter zone normally comprises the part of the U-part between the first 105 and the second 106 pressure controlling device and the second zone preferably comprises the top part 104 and optionally the upper part of the up-flow leg. The second pressure controlling device 106 aids in maintaining the applied pressure in the first zone and reduces the pressure in the circulating broth before entering the second zone. The increased pressure in the first zone in the fermenter raises the mass transfer from the gas phase and into the liquid phase and hereby increases the concentration/amount of the gaseous substrate(s) available to the microorganisms. When the second pressure device 106 reduces the pressure in the second zone, the solubility of the gases in the fermentation broth is reduced. This increases the mass transfer of solubilized gaseous components from the broth and into the gas phase and improves the coalescence of gas bubbles. As a result, separation of waste gases in the top part is improved significantly.

According to an alternative embodiment of the method, the fermenter may be equipped with three or more pressure controlling devices. The second pressure controlling device 106 re-establishes pressure losses occurring through the fermenter, e.g. in the mixers, followed by a third pressure controlling device 108 which reduces the pressure in the following zone comprising the top part 104 of the fermenter. The second pressure controlling device 106 may also decrease the pressure slightly in the fermenter, which is then further decreased by the third pressure controlling device 108 before the fermentation broth enters the top part 104. In the latter embodiment, the fermenter is divided into three zones in which the pressure is reduced in two steps before the broth enters the top part 104. This two-step reduction of the pressure further improves the mass transfer of gases from the broth and coalescence of gas bubbles and hereby increases separation of waste gases in the top part 104.

The pressure is mainly controlled in the different zones by the computer using input from the pressure sensors in the fermenter. The first pressure controlling device 105 applies a pressure to the broth, which is up to 8 bars or more and preferably 0.5-5 bars. The hydrostatic pressure increases the pressure even further through the down-flow leg and decreases the pressure correspondingly during passage through the up-flow leg of the fermenter. The contribution of the hydrostatic pressure depends on the height of fermenter. If the pressure is reduced by applying a suction or vacuum in the top part 104 during fermentation, e.g. for assisting removal of waste gases, the absolute pressure applied in the first zone is reduced correspondingly. The pressure difference between the pressure applied by the first pressure controlling device 105 in the first zone (i.e. the U-part) and the pressure in the top part 104 should preferably be 0.5-5 bars.

Gas separation in the top part may be improved by flushing the headspace above the liquid surface in the top part using a flushing or stripping gas. The flushing gas is introduced co-currently, con-currently or cross-currently to the liquid flow in the top part. Alternatively, the flushing gas may be introduced into the top part below the liquid surface. The flushing/stripping gas then bubbles through the fermentation liquid and strips of gases entrained in the liquid.

The temperature may be regulated during start and fermentation by adding a heating or cooling medium to the heating/cooling jacket 121.

The pressure and other variables are normally controlled in relation to setpoints or intervals in process control systems normally provided by the skilled person. The data from other sensors in the fermenter, and optionally in the recirculation supernatant line, are included in the control of parameters, e.g. pressure, temperature, substrate gas addition, pH adjustment, addition of make-up water, aqueous substrate media and/or recirculation of supernatant, in the system using e.g. Process Analytical Technology (PAT) and/or multivariate data analysis combined with multivariate process control.

During operation, the fermentation process normally reaches a steady state in which the majority of variables, including productivity, is essentially stable (i.e. varying less than 50 % between three measurements, and preferably less than 10 % between five measurements, taken half a residence time apart (where residence time is due to dilution rate).

Ideally, the fermenter may be run continuously for an extended period of time, e.g. many months without being shut down, but it is preferred to run the fermenter for a month before shutting down. Downstream processing of the fermentation liquid extracted from the fermenter is essentially similar to the process described in EP 1183326 B. Liquid fractions (supernatant), having a low content of biomass/product substances, from the first separator 35, the sterilization unit 39 and the second separator 43 are drained off through conduits 36,46,47, respectively, and are returned at 122 to the fermenter, e.g. after a short heat treatment, via the recirculation conduit 25. One or more of the fermenters according to the invention may be connected to a common downstream processing system.

A spray drying unit may be omitted in downstream processing, if the product is to be used as a liquid concentrate. Optionally, the sterilizing unit 39 may also be omitted on a part of or on the entire product stream.

| Ref. no. | Ref. name |
|---|---|
| 1 | Mixer unit |
| 2 | Position lockers |
| 3 | Through openings |
| 4 | Frame |
| 5 | Rod |
| 6 | Static mixer element according to the invention |

(continued)

| Ref. no. | Ref. name |
|---|---|
| 7 | Static mixer element according to prior art |
| 8 | Fastening means fixing rod to frame |
| 100 | U-shaped fermenter |
| 101 | Down-stream leg |
| 102 | Up-stream leg |
| 103 | Connecting part |
| 104 | Top part |
| 105 | Pressure controlling means such as a pump |
| 106 | Pressure controlling means |
| 107 | Head space |
| 108 | Liquid surface |
| 109 | Inlet to top part |
| 110 | Inlet or injection point for gas |
| 111 | Temperature regulating device |
| 112 | Inlet to top part |
| 113 | Outlet |
| 114 | Connection part between top part and up- and down-legs |
| 115 | Flow modifying means in top part |
| 116 | Static mixer |

**Claims**

1. A static mixer element configured to reduce the size of gas bubbles in a liquid for installation in a flow channel which static mixer element comprises at least two mixer units (1) extending over a cross-section area of the flow channel which mixer units (1) comprise a plurality of through openings (3), the static mixer element comprises a first mixer unit (1) and a second and adjacent mixer unit (1), which first and second mixer units are displaced relative to each other in the direction of flow, in such a way that a through opening (3) in the first mixer unit (1) at least partly faces a surface of the second and adjacent mixer unit (1) thereby forcing the fluid flow to change direction

    wherein adjacent mixer units (1) have substantially the same height or thickness h, and are substantially parallel having a distance d between them, which distance d may be

        - approximately 0 as two adjacent mixer units (1) may be in contact with each other at one or more points, or
        - the distance d may be larger than 0 and may be at least 0.5 the thickness of a mixer unit (1) i.e. 0.5xh, or the distance d may be at least the thickness of a mixer unit i.e. 1xh, or
        - the distance d may be at maximum 10 times the thickness of a mixer unit (1) i.e. 10xh, or at maximum 5 times the thickness of a mixer unit i.e. 5xh, or at maximum 3 times the thickness of a mixer unit i.e. 3xh,

    wherein the mixer units (1) are positioned in and attached to a frame (4), which frame defines a position of each mixer units (1) relative to all other mixer units in a static mixer element,
    wherein each mixer unit (1) comprises a plurality of position lockers (2), the position lockers (2) being distributed equally, i.e. with same radial distance between adjacent position lockers (2) measured in degrees (°), along a circle with radius r and centre C,
    **characterized in** a plurality of rods (5) extending in the full length of the static mixer element and fitting into openings constituting the position lockers (2),
    wherein the rods (5) are fastened to the frame (4) by fastening means (8).

EP 4 072 717 B1

2. A static mixer element according to claim 1, wherein the distance d between adjacent mixer units is at maximum 0.030 m, or at maximum 0.020 m or at maximum 0.010 m.

3. A static mixer element according to any previous claim, wherein the static mixer element comprises at least two mixer units (1), e.g. exactly or at least 3, 4, 5, 6, 7, 8, 9, 10 or more mixer units (1), or between 2 and 100 mixer units, or between 2 and 50 mixer units or between 4 and 40 mixer units or between 4 and 35 mixer units, where each through opening (3) in each first mixer unit (1) at least partly faces a surface of a following mixer unit (1) thereby forcing the fluid flow to change direction.

4. A static mixer element according to any previous claim, wherein each mixer unit (1) forms a fluid-tight connection to or lies against an inner surface of the flow channel.

5. A static mixer element according to any previous claim, wherein a first and a following mixer unit (1) are substantially parallel and comprising a surface facing perpendicular to the direction of flow, i.e. a surface of a first mixer unit (1) facing a surface of a following mixer units deviates less than 10°, normally less than 5° or less than 2° from the following mixer unit (1).

6. A static mixer element according to any previous claim, wherein the through going openings (3) in each mixer unit (1) are of same shape, and size and the shape of the through going openings (3) are e.g. rhombic or circular or rectangular.

7. A static mixer section comprising a plurality of blocks of static mixers where at least one block of static mixers has the function of distributing gas bubbles over the flow cross-section and at least one block of static mixers is constituted of two or more static mixer elements according to any one of claims 1-6.

8. A reactor comprising a static mixer element according to any of the previous claims 1-6, wherein each static mixer element of the reactor (100) is positioned in a flow channel or a tubular part being part of the reactor, which flow channel or tubular part comprises a cavity comprising an inlet opening and an outlet opening, optionally the reactor is a loop reactor.

9. A reactor according to claim 8, wherein the size of the cross-sectional area of the flow channel or tubular part of the reactor varies less than 10%, e.g. less than 5% compared to the inlet of the flow channel or tubular reactor.

10. Use of a static mixer element according to any of the claims 1-6 in a reactor for mixing a two-phased flow comprising a liquid and a gas to be mixed at a pressure between 1-10 bar, e.g. between 2-8 bar, e.g. at a pressure between 2 and 6 bar, e.g. at a pressure above 1 bar or above 2 bar, or above 3 bar, or e.g. at a pressure below 7 bar.

**Patentansprüche**

1. Statisches Mischelement, welches dafür eingerichtet ist, die Größe von Gasblasen in einer Flüssigkeit zu verringern, zum Einbauen in einen Strömungskanal, das statische Mischelement aufweisend mindestens zwei Mischereinheiten (1), welche sich über eine Querschnittsfläche des Strömungskanals erstrecken, die Mischereinheiten (1) jeweils aufweisend eine Mehrzahl von Durchgangsöffnungen (3), das statische Mischelement aufweisend eine erste Mischereinheit (1) und eine zweite, benachbarte Mischereinheit (1), wobei die erste Mischereinheit und die zweite Mischereinheit in der Strömungsrichtung derart gegeneinander versetzt angeordnet sind, dass eine Durchgangsöffnung (3) in der ersten Mischereinheit (1) mindestens teilweise einer Fläche der zweiten, benachbarten Mischereinheit (1) zugewandt ist, wodurch die Fluidströmung zum Ändern ihrer Richtung gezwungen wird,

wobei benachbarte Mischereinheiten (1) im Wesentlichen dieselbe Höhe oder Dicke h aufweisen und mit einem Abstand d dazwischen im Wesentlichen parallel zueinander angeordnet sind, wobei der Abstand d

- ungefähr 0 betragen kann, da zwei benachbarte Mischereinheiten (1) an einem oder mehreren Punkte miteinander in Kontakt stehen können, oder
- der Abstand d größer als 0 sein kann und mindestens das 0,5-fache der Dicke einer Mischereinheit (1) betragen kann, das heißt 0,5xh, oder der Abstand d mindestens gleich der Dicke einer Mischereinheit (1) sein kann, das heißt 1xh, oder
- der Abstand d höchstens das 10-fache der Dicke einer Mischereinheit (1) betragen kann, das heißt 10xh, oder höchstens das 5-fache der Dicke einer Mischereinheit betragen kann, das heißt 5xh, oder höchstens das 3-fache der Dicke einer Mischereinheit betragen kann, das heißt 3xh,

wobei die Mischereinheiten (1) in einem Rahmen (4) angeordnet und an diesem angebracht sein können, wobei der Rahmen eine Position jeder der Mischereinheiten (1) relativ zu allen übrigen Mischereinheiten in einem statischen Mischelement definiert,
wobei jede der Mischereinheit (1) eine Mehrzahl von Positionsfixierungen (2) aufweist, welche gleichmäßig verteilt sind, das heißt mit demselben Radialabstand gemessen in Grad (°) zwischen benachbarten Positionsfixierungen (2) entlang eines Kreises mit dem Radius r und dem Mittelpunkt C,
**gekennzeichnet durch**,
eine Mehrzahl von Stäben (5), welche sich über die gesamte Länge des statischen Mischelements erstrecken und in Öffnungen passen, welche die Positionsfixierungen (2) darstellen,
wobei die Stäbe (5) durch Befestigungsmittel (8) am Rahmen (4) befestigt sind.

2. Statisches Mischelement nach Anspruch 1, wobei der Abstand d zwischen benachbarten Mischereinheiten höchstens 0,03 m oder höchstens 0,020 m oder höchstens 0,010 m beträgt.

3. Statisches Mischelement nach einem der vorstehenden Ansprüche, wobei das statische Mischelement mindestens zwei Mischereinheiten (1) aufweist, wobei es zum Beispiel genau oder mindestens 3, 4, 5, 6, 7, 8, 9, 10 oder mehr Mischereinheiten (1) aufweist, oder zwischen 2 und 100 Mischereinheiten oder zwischen 2 und 50 Mischereinheiten oder zwischen 4 und 40 Mischereinheiten oder zwischen 4 und 35 Mischereinheiten aufweist, wobei jede Durchgangsöffnung (3) in jeder ersten Mischereinheit (1) mindestens teilweise einer Fläche einer darauffolgenden Mischereinheit (1) zugewandt ist, wodurch die Fluidströmung zum Ändern ihrer Richtung gezwungen wird.

4. Statisches Mischelement nach einem der vorstehenden Ansprüche, wobei jede der Mischereinheiten (1) eine fluiddichte Verbindung mit einer Innenfläche des Strömungskanals bildet oder an einer solchen Innenfläche anliegt.

5. Statisches Mischelement nach einem der vorstehenden Ansprüche, wobei eine erste Mischereinheit und eine darauffolgende Mischereinheit (1) im Wesentlichen parallel zueinander angeordnet sind und eine Fläche aufweisen, welche lotrecht zur Strömungsrichtung ausgerichtet ist, das heißt, eine Fläche einer ersten Mischereinheit (1), welche einer Fläche einer darauffolgenden Mischereinheit zugewandt ist, weicht um weniger als 10°, üblicherweise um weniger als 5° oder weniger als 2°, von der darauffolgenden Mischereinheit (1) ab.

6. Statisches Mischelement nach einem der vorstehenden Ansprüche, wobei die durchgehenden Öffnungen (3) in jeder der Mischereinheiten (1) dieselbe Form und dieselbe Größe aufweisen und die Form der durchgehenden Öffnungen (3) zum Beispiel rautenförmig oder kreisförmig oder rechteckig ist.

7. Statischer Mischerabschnitt, welcher eine Mehrzahl von Blöcken statischer Mischer aufweist, wobei mindestens ein Block statischer Mischer die Funktion umfasst, Gasblasen über den Strömungsquerschnitt zu verteilen, und mindestens ein Block statischer Mischer aus einem oder mehreren statischen Mischelementen nach einem der Ansprüche 1 - 6 gebildet ist.

8. Reaktor aufweisend ein statisches Mischelement nach einem der vorstehenden Ansprüche 1 - 6, wobei jedes der statischen Mischelemente des Reaktors (100) in einem Strömungskanal oder einem rohrförmigen Teil, welcher einen Bestandteil des Reaktors bildet, angeordnet ist, wobei der Strömungskanal oder der rohrförmige Teil einen Hohlraum, welcher eine Einlassöffnung und eine Auslassöffnung aufweist, aufweist, wobei der Reaktor wahlweise ein Schleifenreaktor ist.

9. Reaktor nach Anspruch 8, wobei die Größe der Querschnittsfläche des Strömungskanals oder des rohrförmigen Teils des Reaktors um weniger als 10 % variiert, zum Beispiel um weniger als 5 % im Vergleich zum Einlass des Strömungskanals oder des rohrförmigen Reaktors.

10. Verwendung eines statischen Mischelements nach einem der Ansprüche 1 - 6 in einem Reaktor zum Mischen einer zweiphasigen Strömung, welche eine Flüssigkeit und ein Gas aufweist, welche bei einem Druck von zwischen 1 und 10 Bar, zum Beispiel bei zwischen 2 und 8 Bar, zum Beispiel bei einem Druck von zwischen 2 und 6 Bar, zum Beispiel bei einem Druck von mehr als 1 Bar oder mehr als 2 Bar oder mehr als 3 Bar, oder bei einem Druck von weniger als 7 Bar, zu vermischen sind.

**Revendications**

1. Élément de mélangeur statique configuré pour réduire la taille de bulles de gaz dans un liquide, destiné à être installé dans un canal d'écoulement, ledit élément de mélangeur statique comprenant au moins deux unités de mélangeur (1) s'étendent sur une aire de section transversale du canal d'écoulement, lesdites unités de mélangeur (1) comprenant une pluralité d'ouvertures traversantes (3), l'élément de mélangeur statique comprenant une première unité de mélangeur (1) et une deuxième unité de mélangeur adjacente (1), lesdites première et deuxième unités de mélangeur étant déplacées l'une par rapport à l'autre dans la direction d'écoulement, de telle façon qu'une ouverture traversante (3) dans la première unité de mélangeur (1) fait face au moins partiellement à une surface de la deuxième unité de mélangeur adjacente (1), forçant ainsi l'écoulement de fluide à changer de direction,

   dans lequel des unités de mélangeur (1) adjacentes présentent substantiellement la même hauteur ou épaisseur h, et sont substantiellement parallèles, avec une distance d entre elles, ladite distance d pouvant être

   - approximativement de 0, sachant que deux unités de mélangeur (1) adjacentes peuvent être en contact l'une avec l'autre en un ou plusieurs points, ou
   - la distance d pouvant être supérieure à 0 et pouvant être au moins égale à 0,5 fois l'épaisseur d'une unité de mélangeur (1), c'est-à-dire 0,5xh, ou la distance d pouvant être au moins égale à l'épaisseur d'une unité de mélangeur, c'est-à-dire 1xh, ou
   - la distance d pouvant être au maximum égale à 10 fois l'épaisseur d'une unité de mélangeur (1), c'est-à-dire 10xh, ou au maximum égale à 5 fois l'épaisseur d'une unité de mélangeur, c'est-à-dire 5xh, ou au maximum égale à 3 fois l'épaisseur d'une unité de mélangeur, c'est-à-dire 3xh,

   dans lequel les unités de mélangeur (1) sont positionnées dans un cadre (4) et fixées à celui-ci, ledit cadre définissant une position de chacune des unités de mélangeur (1) par rapport à toutes les autres unités de mélangeur dans un élément de mélangeur statique,
   dans lequel chaque unité de mélangeur (1) comprend une pluralité de verrous de position (2),
   les verrous de position (2) étant répartis uniformément, c'est-à-dire avec la même distance radiale entre des verrous de position (2) adjacents, mesurée en degrés (°), le long d'un cercle avec un rayon r et un centre C,
   **caractérisé par**
   une pluralité de tiges (5) s'étendant sur toute la longueur de l'élément de mélangeur statique et s'ajustant dans des ouvertures constituant les verrous de position (2),
   dans lequel les tiges (5) sont fixées au cadre (4) par des moyens de fixation (8).

2. Élément de mélangeur statique selon revendication 1, dans lequel la distance d entre des unités de mélangeur adjacentes mesure au maximum 0,030 m, ou au maximum 0,020 m ou au maximum 0,010 m.

3. Élément de mélangeur statique selon l'une quelconque des revendications précédentes, dans lequel l'élément de mélangeur statique comprend au moins deux unités de mélangeur (1), par exemple exactement ou au moins 3, 4, 5, 6, 7, 8, 9, 10 unités de mélangeur (1) ou plus, ou entre 2 et 100 unités de mélangeur, ou entre 2 et 50 unités de mélangeur ou entre 4 et 40 unités de mélangeur ou entre 4 et 35 unités de mélangeur, dans lequel chaque ouverture traversante (3) dans chaque première unité de mélangeur (1) fait au moins partiellement face à une surface d'une unité de mélangeur (1) suivante, forçant ainsi l'écoulement de fluide à changer de direction.

4. Élément de mélangeur statique selon l'une quelconque des revendications précédentes, dans lequel chaque unité de mélangeur (1) forme une liaison étanche au fluide ou repose contre une surface intérieure du canal d'écoulement.

5. Élément de mélangeur statique selon l'une quelconque des revendications précédentes, dans lequel une première unité de mélangeur (1) et une suivante sont substantiellement parallèles et comprennent une surface faisant face perpendiculairement à la direction d'écoulement, c'est-à-dire qu'une surface d'une première unité de mélangeur (1) faisant face à une surface d'une unité de mélangeur suivante dévie de moins de 10°, normalement de moins de 5° ou moins de 2° par rapport à l'unité de mélangeur (1) suivante.

6. Élément de mélangeur statique selon l'une quelconque des revendications précédentes, dans lequel les ouvertures traversantes (3) dans chaque unité de mélangeur (1) présentent la même forme et taille et la forme des ouvertures traversantes (3) est rhombique ou circulaire ou rectangulaire.

7. Section de mélangeur statique comprenant une pluralité de blocs de mélangeurs statiques, dans laquelle au moins un bloc de mélangeurs statiques sert à répartir des bulles de gaz sur la section transversale d'écoulement et au moins un bloc de mélangeurs statiques est constitué de deux ou plusieurs éléments de mélangeur statique selon l'une quelconque des revendications 1 à 6.

8. Réacteur comprenant un élément de mélangeur statique selon l'une quelconque des revendications précédentes 1 à 6, dans lequel chaque élément de mélangeur statique du réacteur (100) est positionné dans un canal d'écoulement ou une partie tubulaire faisant partie du réacteur, ledit canal d'écoulement ou ladite partie tubulaire comprenant une cavité comprenant une ouverture d'entrée et une ouverture de sortie, le réacteur étant optionnellement un réacteur à boucle.

9. Réacteur selon la revendication 8, dans lequel la taille de l'aire de section transversale du canal d'écoulement ou de la partie tubulaire varie de moins de 10 %, par exemple de moins de 5 %, en comparaison avec l'entrée du canal d'écoulement ou du réacteur tubulaire.

10. Utilisation d'un élément de mélangeur statique selon l'une quelconque des revendications 1 à 6 dans un réacteur pour le mélange d'un écoulement biphasé comprenant un liquide et un gaz destinés à être mélangés à une pression entre 1 et 10 bar, par exemple entre 2 et 8 bar, par exemple à une pression entre 2 et 6 bar, par exemple à une pression supérieure à 1 bar ou supérieure à 2 bar, ou supérieure à 3 bar, ou par exemple à une pression inférieure à 7 bar.

Fig. 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4062524 A
- US 2011244543 A1
- US 2017056846 A1

- KR 2017009397
- US 20170205070 A1
- EP 1183326 B